(19) Europäisches Patentamt
European Patent Office
Office européen des brevets

(11) EP 4 667 470 A1

(12) **EUROPEAN PATENT APPLICATION**
published in accordance with Art. 153(4) EPC

(43) Date of publication:
**24.12.2025 Bulletin 2025/52**

(21) Application number: **24756161.6**

(22) Date of filing: **06.02.2024**

(51) International Patent Classification (IPC):
***C07D 487/10*** *(2006.01)* ***A61K 31/407*** *(2006.01)*
***A61P 35/00*** *(2006.01)*

(52) Cooperative Patent Classification (CPC):
**A61K 31/407; A61P 35/00; C07D 487/10**

(86) International application number:
**PCT/CN2024/076332**

(87) International publication number:
**WO 2024/169788 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.02.2023 CN 202310103600**

(71) Applicant: **Hitgen Inc.**
**Chengdu, Sichuan 610200 (CN)**

(72) Inventors:
- **LI, Jin**
  **Chengdu, Sichuan 610200 (CN)**
- **LIU, Chuan**
  **Chengdu, Sichuan 610200 (CN)**
- **XIA, Shuai**
  **Chengdu, Sichuan 610200 (CN)**
- **DONG, Xiaofei**
  **Chengdu, Sichuan 610200 (CN)**
- **XIANG, Sichuan**
  **Chengdu, Sichuan 610200 (CN)**
- **LIAO, Hui**
  **Chengdu, Sichuan 610200 (CN)**
- **DOU, Dengfeng**
  **Chengdu, Sichuan 610200 (CN)**
- **CHEN, Qiuxia**
  **Chengdu, Sichuan 610200 (CN)**

(74) Representative: **De Clercq & Partners**
**Edgard Gevaertdreef 10a**
**9830 Sint-Martens-Latem (BE)**

# (54) FUSED RING COMPOUND AND USE THEREOF

(57) The present invention provides a compound as shown in formula I, having a binding effect with E3 ligase protein CRBN, and a use of the compound in the preparation of a medicine for treating abnormal cell proliferation diseases.

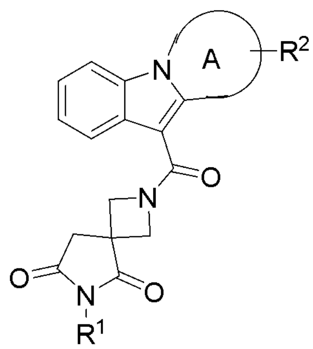

Formula I

EP 4 667 470 A1

## Description

### FIELD

The present disclosure belongs to the field of medicine, and in particular relates to a novel spirocyclic ligand compound having a binding effect with cereblon E3 ubiquitin ligase protein.

### BACKGROUND

Protein degradation is a highly regulated and necessary process for maintaining cell homeostasis. The selective identification and removal of damaged, misfolded or excessive protein are achieved through ubiquitin-proteasome pathway (UPP). UPP is responsible for the removal of defective proteins, which is characterized by ATP dependence, high efficiency, and high selectivity, and its catalytic part is the ubiquitin ligase E3, but the proteins to be degraded need to be recruited first. PROTACs technology is designed based on UPP principle, which connects the ligand of target protein with the ligand of E3 ligase by a suitable chemical bond, so that the target protein can be recognized and the binding ability of E3 ligase with target protein can be enhanced, and then the target protein can be targeted for ubiquitination and forced to be degraded, with the characteristics of catalyst amount, high efficiency, high selectivity, and the like.

Protein is labeled by multiple ubiquitin molecules through covalent linkage between E3 ubiquitin ligase and terminal lysine residue for proteasome degradation, in which the protein was digested into small peptides and finally into its constituent amino acids, and the amino acids are used as building blocks of new protein. Defective proteasome degradation is related to many clinical diseases, including Alzheimer's disease, Parkinson's disease, Huntington's disease, muscular dystrophy, cardiovascular disease, cancer, and the like.

Cereblon, a thalidomide-binding protein, is a part of E3 ubiquitin ligase protein complex, which acts as a substrate receptor and selectively acts on ubiquitinated protein. Cereblon is a protein encoded by human CRBN gene and forms E3 ubiquitination ligase complex together with damaged DNA binding protein 1 (DDBI), Cullin-4A (CUL4A) and regulator of Cullin-1 (ROCI). The complex can ubiquitinate a series of proteins, although the specific mechanism is not clear. Cereblon is an E3 ligase known to be commonly used in PROTACs technology.

The present disclosure discloses novel spirocyclic compounds, which can be used as effective CRBN ligands, and can be used to further synthesize corresponding PROTACs bifunctional compounds which can degrade proteins and target chimeras, and can be used for treating various medical diseases, especially abnormal proliferation of cells.

### SUMMARY

The present disclosure provides a compound represented by Formula I, or a stereoisomer, or a deuterated compound, or a pharmaceutically acceptable salt thereof:

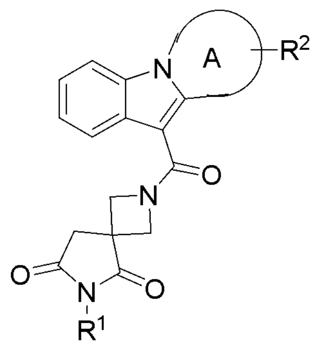

Formula I

wherein,

$R^1$ is selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring);

ring A is selected from the group consisting of 4- to 12-membered heterocycloalkyl and 5- to 10-membered heteroaromatic ring; wherein the heterocycloalkyl or heteroaromatic ring can be further optionally substituted by one, two, three or four independent $R^{A1}$;

each $R^{A1}$ is independently selected from the group consisting of hydrogen, halogen, cyano, nitro, =O, =S, $=CR^{A2}R^{A3}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{A2}$, $-C_{0-4}$ alkylene-$OC(O)R^{A2}$, $-C_{0-4}$ alkylene-$SR^{A2}$, $-C_{0-4}$ alkylene-$S(O)_2R^{A2}$, $-C_{0-4}$ alkylene-$S(O)R^{A2}$, $-C_{0-4}$ alkylene-$S(O)_2NR^{A2}R^{A3}$, $-C_{0-4}$ alkylene-$S(O)NR^{A2}R^{A3}$, $-C_{0-4}$ alkylene-$C(O)R^{A2}$, $-C_{0-4}$ alkylene-$C(O)OR^{A2}$, $-C_{0-4}$ alkylene-$C(O)NR^{A2}R^{A3}$, $-C_{0-4}$ alkylene-$NR^{A2}R^{A3}$, $-C_{0-4}$ alkylene-$NR^{A2}C(O)R^{A3}$, $-C_{0-4}$ alkylene-$NR^{A2}S(O)_2R^{A3}$, $-C_{0-4}$ alkylene-$NR^{A2}S(O)R^{A3}$, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring); wherein the alkylene, cycloalkyl, heterocycloalkyl, aromatic ring or heteroaromatic ring can be further optionally substituted by one, two, three or four independent $R^{A4}$;

each $R^{A4}$ is independently selected from the group consisting of hydrogen, halogen, cyano, nitro, =O, =S, $=CR^{A2}R^{A3}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{A2}$, $-C_{0-4}$ alkylene-$OC(O)R^{A2}$, $-C_{0-4}$ alkylene-$SR^{A2}$, $-C_{0-4}$ alkylene-$S(O)_2R^{A2}$, $-C_{0-4}$ alkylene-$S(O)R^{A2}$, $-C_{0-4}$ alkylene-$S(O)_2NR^{A2}R^{A3}$, $-C_{0-4}$ alkylene-$S(O)NR^{A2}R^{A3}$, $-C_{0-4}$ alkylene-$C(O)R^{A2}$, $-C_{0-4}$ alkylene-$C(O)OR^{A2}$, $-C_{0-4}$ alkylene-$C(O)NR^{A2}R^{A3}$, $-C_{0-4}$ alkylene-$NR^{A2}R^{A3}$, $-C_{0-4}$ alkylene-$NR^{A2}C(O)R^{A3}$, $-C_{0-4}$ alkylene-$NR^{A2}S(O)_2R^{A3}$ and $-C_{0-4}$ alkylene-$NR^{A2}S(O)R^{A3}$;

$R^{A2}$ and $R^{A3}$ are independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

$R^2$ is selected from the group consisting of hydrogen, halogen, cyano, nitro, =O, =S, $=CR^{21}R^{22}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{21}$, $-C_{0-4}$ alkylene-$OC(O)R^{21}$, $-C_{0-4}$ alkylene-$SR^{21}$, $-C_{0-4}$ alkylene-$S(O)_2R^{21}$, $-C_{0-4}$ alkylene-$S(O)R^{21}$, $-C_{0-4}$ alkylene-$S(O)_2NR^{21}R^{22}$, $-C_{0-4}$ alkylene-$S(O)NR^{21}R^{22}$, $-C_{0-4}$ alkylene-$C(O)R^{21}$, $-C_{0-4}$ alkylene-$C(O)OR^{21}$, $-C_{0-4}$ alkylene-$C(O)NR^{21}R^{22}$, $-C_{0-4}$ alkylene-$NR^{21}R^{22}$, $-C_{0-4}$ alkylene-$NR^{21}C(O)R^{22}$, $-C_{0-4}$ alkylene-$NR^{21}S(O)_2R^{22}$, $-C_{0-4}$ alkylene-$NR^{21}S(O)R^{22}$, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring); wherein the alkylene, cycloalkyl, heterocycloalkyl, aromatic ring or heteroaromatic ring can be further optionally substituted by one, two, three or four independent $R^{23}$;

each $R^{23}$ is independently selected from the group consisting of hydrogen, halogen, cyano, nitro, =O, =S, $=CR^{21}R^{22}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{21}$, $-C_{0-4}$ alkylene-$OC(O)R^{21}$, $-C_{0-4}$ alkylene-$SR^{21}$, $-C_{0-4}$ alkylene-$S(O)_2R^{21}$, $-C_{0-4}$ alkylene-$S(O)R^{21}$, $-C_{0-4}$ alkylene-$S(O)_2NR^{21}R^{22}$, $-C_{0-4}$ alkylene-$S(O)NR^{21}R^{22}$, $-C_{0-4}$ alkylene-$C(O)R^{21}$, $-C_{0-4}$ alkylene-$C(O)OR^{21}$, $-C_{0-4}$ alkylene-$C(O)NR^{21}R^{22}$, $-C_{0-4}$ alkylene-$NR^{21}R^{22}$, $-C_{0-4}$ alkylene-$NR^{21}C(O)R^{22}$, $-C_{0-4}$ alkylene-$NR^{21}S(O)_2R^{22}$, $-C_{0-4}$ alkylene-$NR^{21}S(O)R^{22}$, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring); wherein the alkylene, cycloalkyl, heterocycloalkyl, aromatic ring or heteroaromatic ring can be further optionally substituted by one, two, three or four independent $R^{26}$;

$R^{21}$ and $R^{22}$ are independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{24}$, $-C_{0-4}$ alkylene-$OC(O)R^{24}$, $-C_{0-4}$ alkylene-$SR^{24}$, $-C_{0-4}$ alkylene-$S(O)_2R^{24}$, $-C_{0-4}$ alkylene-$S(O)R^{24}$, $-C_{0-4}$ alkylene-$S(O)_2NR^{24}R^{25}$, $-C_{0-4}$ alkylene-$S(O)NR^{24}R^{25}$, $-C_{0-4}$ alkylene-$C(O)R^{24}$, $-C_{0-4}$ alkylene-$C(O)OR^{24}$, $-C_{0-4}$ alkylene-$C(O)NR^{24}R^{25}$, $-C_{0-4}$ alkylene-$NR^{24}R^{25}$, $-C_{0-4}$ alkylene-$NR^{24}C(O)R^{25}$, $-C_{0-4}$ alkylene-$NR^{24}S(O)_2R^{25}$, $-C_{0-4}$ alkylene-$NR^{24}S(O)R^{25}$, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring); wherein the alkylene, cycloalkyl, heterocycloalkyl, aromatic ring or heteroaromatic ring can be further optionally substituted by one, two, three or four independent $R^{26}$;

each $R^{26}$ is independently selected from the group consisting of hydrogen, halogen, cyano, nitro, =O, =S, $=CR^{24}R^{25}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{24}$, $-C_{0-4}$ alkylene-$OC(O)R^{24}$, $-C_{0-4}$ alkylene-$SR^{24}$, $-C_{0-4}$ alkylene-$S(O)_2R^{24}$, $-C_{0-4}$ alkylene-$S(O)R^{24}$, $-C_{0-4}$ alkylene-$S(O)_2NR^{24}R^{25}$, $-C_{0-4}$ alkylene-$S(O)NR^{24}R^{25}$, $-C_{0-4}$ alkylene-

C(O)$R^{24}$, -$C_{0-4}$ alkylene-C(O)O$R^{24}$, -$C_{0-4}$ alkylene-C(O)N$R^{24}R^{25}$, -$C_{0-4}$ alkylene-N$R^{24}R^{25}$, -$C_{0-4}$ alkylene-N$R^{24}$C(O)$R^{25}$, -$C_{0-4}$ alkylene-N$R^{24}S(O)_2R^{25}$, -$C_{0-4}$ alkylene-N$R^{24}$S(O)$R^{25}$, -$C_{0-4}$ alkylene-(3- to 10-membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring can be further optionally substituted by one, two, three or four independent $R^{27}$;

$R^{24}$ and $R^{25}$ are independently selected from the group consisting of hydrogen, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl; and

each $R^{27}$ is independently selected from the group consisting of hydrogen, halogen, cyano, nitro, =O, =S, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl.

Preferably, $R^1$ is selected from the group consisting of hydrogen and -$C_{1-3}$ alkyl. Specifically, $R^1$ is selected from the group consisting of hydrogen, methyl, ethyl, n-propyl and isopropyl.

Preferably, $R^2$ is selected from the group consisting of hydrogen, halogen, cyano, nitro, =O, =S, -$C_{1-3}$ alkyl, -$C_{2-6}$ alkenyl, -$C_{2-6}$ alkynyl, halogen-substituted -$C_{1-3}$ alkyl, halogen-substituted -$C_{2-6}$ alkenyl and halogen-substituted -$C_{2-6}$ alkynyl. Specifically, $R^2$ is selected from the group consisting of hydrogen, fluorine, chlorine, bromine, cyano, nitro, =O, =S, methyl, ethyl, n-propyl, isopropyl, vinyl, ethynyl, monofluoromethyl, difluoromethyl and trifluoromethyl.

Preferably, the ring A is selected from the group consisting of 5-membered heterocycloalkyl and 6-membered heterocycloalkyl; wherein the heterocycloalkyl can be further optionally substituted by one, two, three or four independent $R^{A1}$,

In some preferred embodiments, the ring A is selected from the group consisting of 5-membered nitrogen-containing heterocycloalkyl and 6-membered nitrogen-containing heterocycloalkyl; wherein the heterocycloalkyl can be further optionally substituted by one, two, three or four independent $R^{A1}$,

In some preferred embodiments, the ring A is selected from the group consisting of 5-membered nitrogen-containing heterocycloalkyl and 6-membered nitrogen-containing heterocycloalkyl, and the nitrogen-containing heterocycloalkyl contains one or two nitrogen atoms; preferably two nitrogen atoms; wherein the heterocycloalkyl can be further optionally substituted by one, two, three or four independent $R^{A1}$; and

each $R^{A1}$ is independently selected from the group consisting of hydrogen, halogen, cyano, nitro, =O, -$C_{1-3}$ alkyl, -$C_{2-6}$ alkenyl, -$C_{2-6}$ alkynyl, halogen-substituted -$C_{1-3}$ alkyl, halogen-substituted -$C_{2-6}$ alkenyl and halogen-substituted -$C_{2-6}$ alkynyl. Specifically, each $R^{A1}$ is selected from the group consisting of hydrogen, fluorine, chlorine, bromine, cyano, nitro, =O, methyl, ethyl, n-propyl, isopropyl, vinyl, ethynyl, monofluoromethyl, difluoromethyl and trifluoromethyl.

More specifically, the ring A is selected from the group consisting of

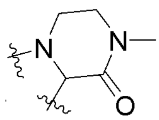,

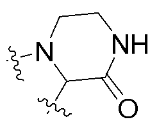, 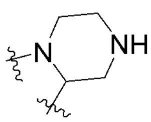, 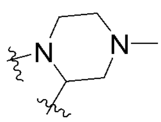, 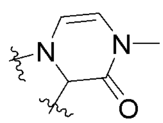, 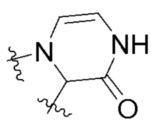, 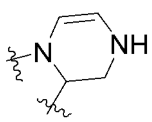,

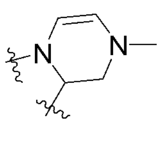, 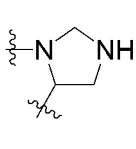, 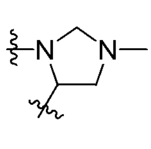, 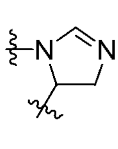, 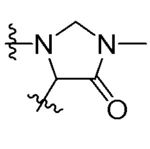, 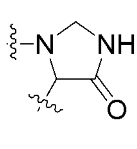 and 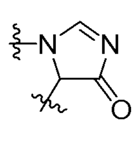.

Preferably, the compound is represented by formula IIA, formula IIB or formula IIC:

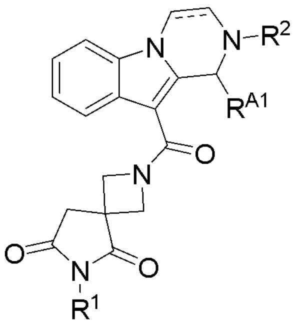

,

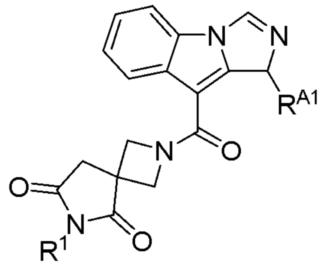

or

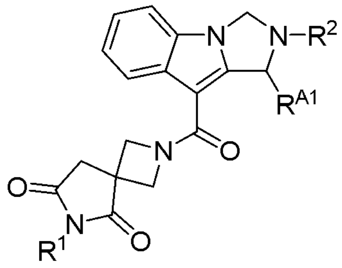

IIA IIB IIC

wherein,

--- is selected from the group consisting of a single bond and a double bond; and

the substituents of $R^1$, $R^{A1}$ and $R^2$ are defined as above.

In some specific embodiments of the present disclosure, the compound is selected from the group consisting of

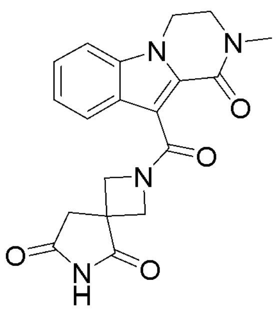

,

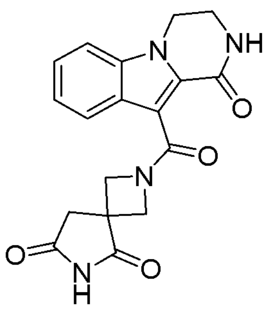

,

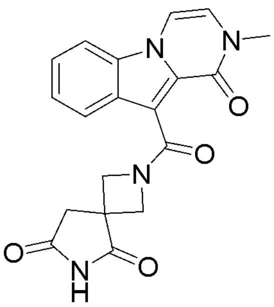

,

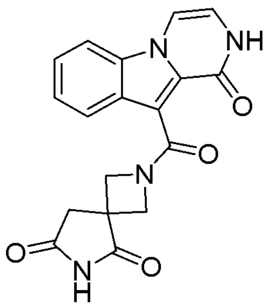

,

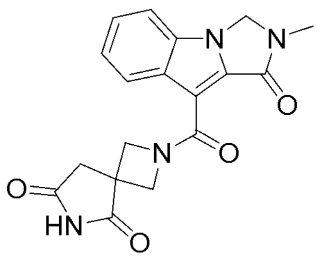

,

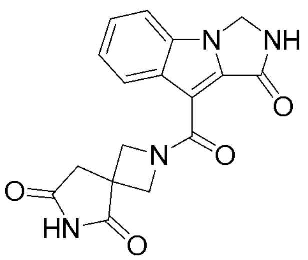

and

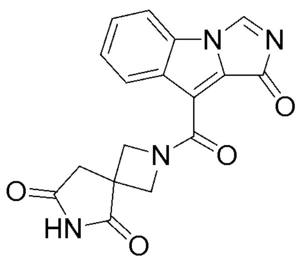

.

The present disclosure further provides use of the above-mentioned compound or the stereoisomer thereof, or the deuterated compound thereof, or the pharmaceutically acceptable salt thereof in the manufacture of a medicament for treating a disease associated with abnormal proliferation of cells.

Further, the disease is cancer.

The present disclosure further provides use of the above-mentioned compound or the stereoisomer thereof, or the deuterated compound thereof, or the pharmaceutically acceptable salt thereof in the manufacture of a medicament for degrading a target protein.

The present disclosure further provides use of the above-mentioned compound or the stereoisomer thereof, or the deuterated compound thereof, or the pharmaceutically acceptable salt thereof as an intermediate in the manufacture of a medicament for degrading a target protein.

The compounds and derivatives provided in the present disclosure can be named according to the IUPAC (International Union of Pure and Applied Chemistry) or CAS (Chemical Abstracts Service, Columbus, OH) nomenclature system.

Definitions of terms used in the present disclosure: Unless otherwise stated, the initial definitions provided for groups or terms herein apply to the groups or terms throughout the specification; for terms that are not specifically defined herein, the meaning thereof should be given by a person skilled in the art based on the disclosure and context

"Substitution" means that a hydrogen atom in the molecule is replaced by other different atoms or molecules. "Substitution" also means that the lone pair of electrons of an atom in a molecule is replaced by =S, =O and the like.

"Further optionally substituted" means that "substitution" may but need not occur, and the expression includes situations where substitution occurs or does not occur.

The minimum and maximum of number of carbon atoms of a hydrocarbon group is indicated by a prefix. For example, the prefix $C_{a-b}$ alkyl indicates any alkyl group containing "a" to "b" carbon atoms. Thus, for example, $C_{1-4}$ alkyl refers to an alkyl group containing 1 to 4 carbon atoms.

"Alkyl" refers to a saturated hydrocarbon chain with a specified number of member atoms. For example, $C_{1-6}$ alkyl refers to an alkyl group with 1 to 6 member atoms, such as 1 to 4 member atoms. Alkyl groups can be linear or branched. Representative branched alkyl groups have one, two or three branches. Alkyl groups may be optionally substituted with one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl) and hexyl. Alkyl groups may also be part of other groups, such as $C_{1-6}$ alkoxy.

"Alkylene" described in the present disclosure refers to a divalent saturated aliphatic hydrocarbon group having a specified number of carbon atoms. "$C_{a-b}$ alkylene" refers to an alkylene group having a to b carbon atoms. Alkylene groups include branched and linear hydrocarbon groups. For example, "$C_{1-6}$ alkylene" is meant to include methylene, ethylene, propylene, 2-methylpropylene, dimethylethylene, pentylene and the like. Therefore, the term "propylene" can be exemplified by the following structure:

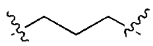.

Similarly, the term "dimethylbutylene" can be exemplified, for example, by any one of the following structures:

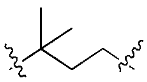 or 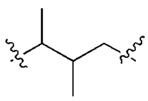.

Furthermore, the term "($C_{1-6}$) alkylene" is meant to include such branched hydrocarbon groups, such as cyclopropylmethylene, which can be exemplified by the following structure:

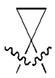.

In addition, for example, -$C_{0-4}$ alkylene can be $C_0$ alkylene, $C_1$ alkylene (such as -$CH_2$-), $C_2$ alkylene (such as -$CH_2CH_2$-), $C_3$ alkylene or $C_4$ alkylene; $C_0$ alkylene means that the group here does not exist and indicates a connection in the form of a chemical bond, for example, A-$C_0$ alkylene-B means A-B, that is, the group A and the group B are directly connected by a chemical bond.

"Alkenyl" refers to a linear or branched hydrocarbon group having a specified number of carbon atoms or 2 to 6 carbon atoms or 2 to 4 carbon atoms in some embodiments and having at least one site of vinyl unsaturation (>C=C<). For example, $C_{a-b}$ alkenyl refers to an alkenyl group having a to b carbon atoms and is intended to include, for example, ethenyl, propenyl, isopropenyl, 1,3-butadienyl, and the like.

The "alkenylene" in the present disclosure refers to a hydrocarbon chain having 2 to 10 carbon atoms, at least one double bond and two unsaturated chemical valences. For example, ($C_3$-$C_6$) alkenylene groups include >C=CH-$CH_2$-, -CH-CH=CH-$CH_2$-, and the like.

"Alkynyl" refers to a linear monovalent hydrocarbon group or a branched monovalent hydrocarbon group containing at least one triple bond. The term "alkynyl" is also intended to include those hydrocarbon groups having one triple bond and one double bond. For example, ($C_2$-$C_6$) alkynyl is intended to include ethynyl, propynyl, and the like.

The "halogen" refers to fluorine, chlorine, bromine or iodine.

The "halogen alkyl" or "halogen-substituted alkyl" refers to an alkyl group in which hydrogen atoms can be replaced by one or more halogen atoms; for example, $C_{1-4}$ halogen alkyl refers to an alkyl group containing 1 to 4 carbon atoms in which hydrogen atoms are replaced by one or more halogen atoms.

The "-OR", "-NRR" and the like described in the present disclosure refers to that group R is connected to the oxygen atom or the nitrogen atom by a single bond.

In "-C(O)R", "-S(O)$_2$R " and the like described in the present disclosure, the oxygen atom is connected to the carbon atom or the sulfur atom by a double bond, and group R is connected to the oxygen atom or the sulfur atom by a single bond.

The "cycloalkyl" and "cycloalkane" described in the present disclosure refer to a saturated or partially saturated cyclic group having multiple carbon atoms and no ring heteroatoms, and having a single ring or multiple rings (including fused, bridged, spiro and adamantane systems). For polycyclic systems with aromatic and nonaromatic rings containing no ring heteroatoms, the term "cycloalkyl" (e.g., 5,6,7,8-tetralin-5-yl) applies when the attachment point is at nonaromatic carbon atoms. The term "cycloalkyl" includes cycloalkenyl groups such as cyclohexenyl. Examples of cycloalkyl groups include, for example, adamantyl, cyclopropyl, cyclobutyl, cyclohexyl, cyclopentyl, cyclooctyl, cyclopentenyl and cyclohexenyl. Examples of cycloalkyl groups including a bicyclic alkyl ring system are dicyclohexyl, dicyclopentyl, dicyclooctyl and the like, such as

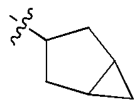,

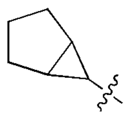 and 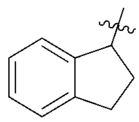.

Adamantyl includes but is not limited to the following structure:

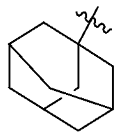.

The "heterocyclic ring", "heterocycloalkyl" and "heterocycloalkane" described in the present disclosure refers to a saturated ring or a non-aromatic unsaturated ring containing at least one heteroatom; wherein the heteroatom refers to a nitrogen atom, an oxygen atom, a sulfur atom, etc. It usually refers to a monovalent saturated or partially unsaturated monocyclic or bicyclic ring system with multiple ring atoms, preferably a monovalent saturated or partially unsaturated monocyclic or bicyclic ring system with 3 to 9 ring atoms, which contains 1, 2 or 3 ring heteroatoms selected from the group consisting of N, O and S, and the remaining ring atoms are carbon. A bicyclic ring is composed of two rings sharing two ring atoms, that is, the bridge separating the two rings is a single bond or a chain of one or two ring atoms. Examples of monocyclic saturated heterocycloalkyl groups are oxetanyl, azetidinyl, pyrrolidinyl, 2-oxo-pyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazinyl, morpholinyl,

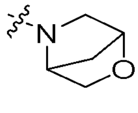,

thiomorpholinyl, 1,1-dioxo-thiomorpholin-4-yl, azepanyl, diazepanyl, homopiperazinyl, or oxaazepanyl. Examples of bicyclic saturated heterocycloalkyl groups are 8-aza-bicyclo[3.2.1]octyl, quinuclidinyl, 8-oxa-3-aza-bicyclo[3.2.1]octyl, 9-aza-bicyclo[3.3.1]nonyl or

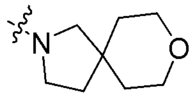.

Examples of partially unsaturated heterocycloalkyl groups are dihydrofuranyl, imidazolinyl, tetrahydropyridyl, or dihydropyranyl.

"Spiro heterocyclic group" and "spiro heterocyclic ring" can be used interchangeably, which refers to a nonaromatic saturated ring or nonaromatic unsaturated ring system with two single rings sharing one carbon atom, and is composed of carbon atoms and heteroatoms selected from the group consisting of nitrogen, oxygen, sulfur and phosphorus atoms. For example, "5- to 12-membered spiro heterocyclic ring" refers to a spiro heterocyclic ring with 5 to 12 ring atoms, of which 1, 2 or 3 ring atoms are heteroatoms.

"Bridged ring" or "bridged ring group" refers to a saturated or unsaturated cyclic group formed by two or more cyclic structures sharing two non-adjacent atoms with each other, and its specific examples include but are not limited to:

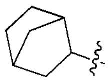, 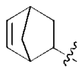, 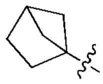

and

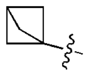.

"Bridged heterocyclic group" and "bridged heterocyclic group" can be used interchangeably, which refers to a saturated or unsaturated cyclic group formed by two or more cyclic structures sharing two non-adjacent atoms with each other, and is composed of carbon atoms and heteroatoms selected from the group consisting of nitrogen, oxygen, sulfur and phosphorus atoms, and its specific examples include but are not limited to:

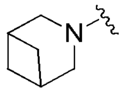,

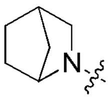, 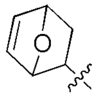, 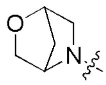 and 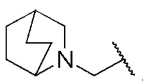.

The "aromatic ring" and "aryl" described in the present disclosure refer to an aromatic hydrocarbon group having multiple carbon atoms. Aryl is generally a monocyclic, bicyclic or tricyclic aryl having 5 to 20 carbon atoms. In addition, the term "aryl" used herein refers to an aromatic substituent that may be a single aromatic ring or multiple aromatic rings fused together. Non-limiting examples include phenyl, naphthyl or tetrahydronaphthyl.

The "heteroaromatic ring" and "heteroaromatic group" described in the present disclosure refer to an aromatic unsaturated ring containing at least one heteroatom, wherein the heteroatom refers to a nitrogen atom, an oxygen atom, a sulfur atom, etc. "Heteroaromatic ring" and "heteroaromatic group" generally refer to aromatic monocyclic or bicyclic hydrocarbons that contain multiple ring atoms, one or more of which are heteroatoms selected from the group consisting of O, N and S, preferably one to three heteroatoms. Heteroaromatic group includes, for example, pyridyl, indolyl, quinoxalinyl, quinolyl, isoquinolinyl, benzothienyl, benzofuranyl, benzothienyl, benzopyranyl, benzothiopyranyl, furyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, or benzoxazolyl.

The "stereoisomer" includes enantiomers and diastereomers.

The "deuterated compound" described in the present disclosure refers to a molecule or group in which one or more hydrogen atoms are replaced by deuterium atoms, wherein the proportion of deuterium atoms is greater than the abundance of deuterium in nature.

The term "pharmaceutically acceptable" means that a carrier, vehicle, diluent, excipient, and/or formed salt is generally chemically or physically compatible with the other ingredients that constitute a pharmaceutical dosage form and physiologically compatible with the receptor.

The terms "salts" and "pharmaceutically acceptable salts" refer to acidic and/or basic salts formed by the above compounds or their stereoisomers with inorganic and/or organic acids and bases, and also include zwitterionic salts (inner salts) as well as quaternary ammonium salts such as alkylammonium salts. These salts can be obtained directly during the final separation and purification of the compounds. They can also be obtained by mixing the above compounds or their stereoisomers, with a certain amount of acid or base appropriately (e.g., equivalent amounts). These salts may be precipitated in the solution and collected by filtration, or recovered after evaporation of the solvent, or obtained by freeze-drying after reaction in an aqueous medium. The salt in the present disclosure can be hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate of the compound.

Apparently, according to the above contents of the present disclosure, in accordance with common technical knowledge and customary means in the art, other various forms of modification, replacement or change may be made without departing from the above basic technical ideas of the present disclosure.

## DETAILED DESCRIPTION

The above contents of the present disclosure are further described in detail below through specific embodiments in the form of examples. However, this should not be understood as the scope of the above subject matter of the present disclosure being limited to the following examples. All technologies realized based on the above contents of the present disclosure belong to the scope of the present disclosure.

The known starting materials of the present disclosure can be synthesized by methods known in the art, or can be purchased from companies such as Energy Chemical, Chengdu Chron Chemicals, Accela ChemBio, and J&K Scientific.

The reagents described in the examples are abbreviated as follows: EDCI: carbodiimide; DMF: N,N- dimethylformamide; ACN: acetonitrile.

Unless otherwise specified in the examples, the reaction is carried out in a nitrogen atmosphere. Unless otherwise specified in the examples, the solution refers to an aqueous solution. Unless otherwise specified in the examples, the reaction temperature is room temperature. Room temperature is the most suitable temperature for reaction, which is 20°C-30°C. Unless otherwise specified in the examples, M is mole per liter.

The structures of the compounds were determined by nuclear magnetic resonance (NMR) and mass spectrometry (MS). NMR shifts (δ) are given in units of 10-6 (ppm). NMR measurements were performed using NMR spectrometers (Bruker Avance III 400 and Bruker Avance 600), and the solvents used were deuterated dimethyl sulfoxide (DMSO-$d_6$), deuterated chloroform ($CDCl_3$) and deuterated methanol (Methol-$d_4$), and the internal standard was tetramethylsilane (TMS). LC-MS measurements were performed using Shimadzu liquid chromatography-mass spectrometry (Shimadzu LC-MS 2020 (ESI)). HPLC measurements were performed using Shimadzu high pressure liquid chromatograph (Shimadzu LC-20A). MPLC (medium pressure liquid chromatography) was performed using Gilson GX-281 reverse phase preparative chromatograph. The silica gel plate used for thin layer chromatography was Yantai Huanghai HSGF254 or Qingdao GF254 silica gel plate, and the specifications of thin layer chromatography used for separating and purifying products were 0.4 mm-0.5 mm. For column chromatography, Yantai Huanghai silica gel 200-300 mesh silica gel was generally used as a carrier.

### Example 1 Preparation of compound A1

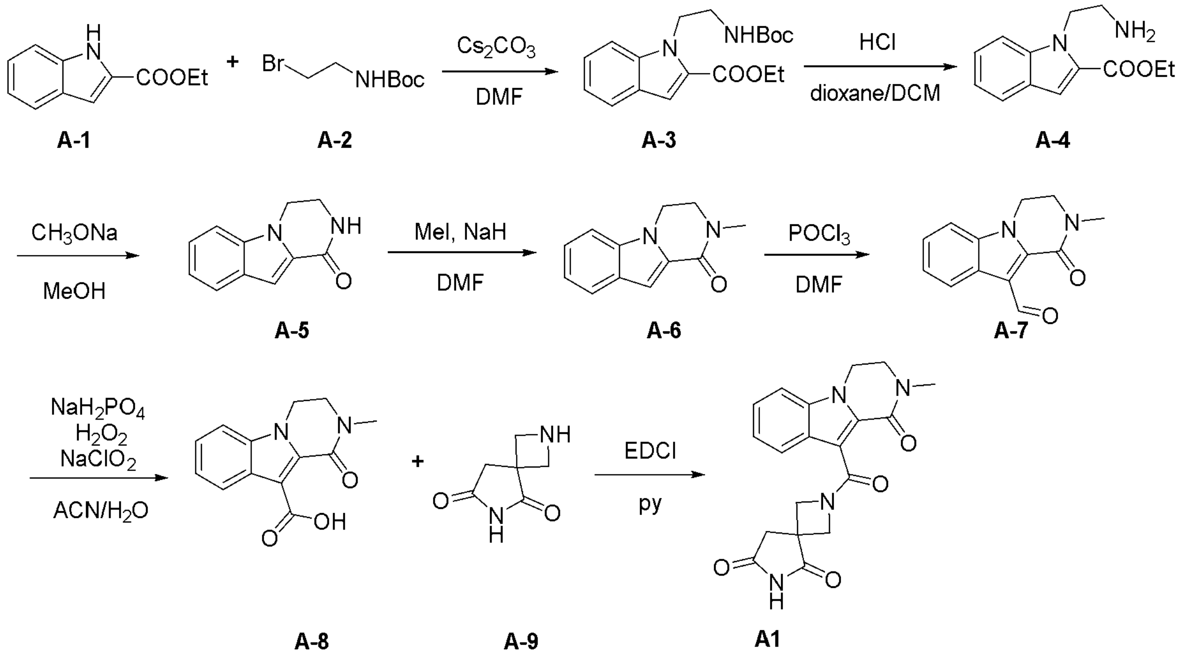


A-1 A-2 A-3 A-4

A-5 A-6 A-7

A-8 A-9 A1

**Step 1: Synthesis of compound A-3**

A-1 (3.9 g, 20.61 mmol), A-2 (9.24 g, 41.22 mmol), cesium carbonate (13.43 g, 41.22 mmol) and DMF (50 mL) were added into a 100 mL reaction flask. The reaction was heated at 100°C overnight until the reaction was completed. The reaction was quenched by adding water and extracted with ethyl acetate (50 mL×3). The organic phase was washed with concentrated brine, dried over anhydrous sodium sulfate and then filtered, the solvent was evaporated to dryness, and the reactant was purified by MPLC to obtain **A-3** (4.1 g, 12.33 mmol, 59.84% yield).

**Step 2 Synthesis of compound A-4**

To a 50 mL reaction flask, **A-3** (4.0 g, 12.03 mmol) and dichloromethane (10 mL) were added, and 10 mL of HCl/dioxane (4M) was added dropwise. The reaction was stirred at room temperature for 1 h, and the solvent was evaporated to dryness to obtain **A-4** (2.6 g, 11.19 mmol, 93.02% yield).

**Step 3 Synthesis of compound A-5**

**A-4** (2.6 g, 11.19 mmol), sodium methoxide (5.82 g, 107.79 mmol) and methanol (20 ml) were added into a 50 mL reaction flask and heated at 65°C overnight until the reaction was completed. The solvent was evaporated to dryness, and the reactant was purified by MPLC to obtain **A-5** (1.8 g, 9.67 mmol, 86.36% yield).

**Step 4 Synthesis of compound A-6**

**A-5 (500** mg, 2.69 mmol), sodium hydride (96.66 mg, 4.03 mmol) and DMF (15 mL) were added into a 50 mL reaction flask. After stirring at room temperature for 30 min, methyl iodide (457.55 mg, 3.22 mmol) was added. The reaction was stirred at room temperature overnight, quenched by adding water and extracted with ethyl acetate (50 mL×3). The organic phase was washed with concentrated brine, dried over anhydrous sodium sulfate and then filtered, and the solvent was evaporated to dryness to obtain **A-6** (500 mg, 2.50 mmol, 93.00% yield).

**Step 5 Synthesis of compound A-7.**

**A-6** (300 mg, 1.50 mmol) and DMF (5 mL) were added into a 50 mL reaction flask. Phosphorus oxychloride (344.59 mg, 2.25 mmol) was added slowly at 0°C, and heated at 60°C overnight, the solvent was evaporated to dryness, and the reactant was purified by MPLC to obtain **A-7** (250 mg, 1.10 mmol, 73.11 % yield).

**Step 6 Synthesis of compound A-8**

A-7 (100 mg, 438.12 μmol), sodium dihydrogen phosphate (78.86 mg, 657.19 μmol), hydrogen peroxide (60.00 μL) and ACN/$H_2O$ (4:1, 3 mL) were added into a 50 mL reaction flask. Sodium chlorite (102.52 mg, 657.19 μmol) was added at 0°C, heated to room temperature and then stirred for 3 h. The reaction solution was filtered and the solvent was evaporated to dryness to obtain **A-8** (90 mg, 368.48 μmol, 84.10% yield).

**Step 7 Synthesis of compound A1**

**A-8** (45 mg, 184.24 μmol), **A-9** (25.82 mg, 184.24 μmol), EDCI (70.64 mg, 368.48 μmol) and pyridine (2 mL) were added into a 50 mL reaction flask. After the reaction was performed overnight at room temperature, the reactant was purified by MPLC to obtain **A1** (10.00 mg, 27.29 μmol, 14.81% yield, 99.6% purity) $^1$H NMR (400 MHz, DMSO-$d_6$) δ 7.64 (d, *J* = 8.0 Hz, 1H), 7.58 (d, *J* = 8.4 Hz, 1H), 7.40-7.36 (m, 1H), 7.24-7.20 (m, 1H), 4.43 - 4.32 (m, 2H), 4.27 (d, *J* = 10.0 Hz, 1H), 4.08 (d, *J* = 10.0 Hz, 1H), 4.00 (d, *J* = 8.8 Hz, 1H), 3.90 (d, *J* = 8.8 Hz, 1H), 3.87 - 3.79 (m, 2H), 3.09 (s, 3H), 3.08 - 2.84 (m, 2H). LC-MS: $[M+H]^+$ $C_{19}H_{19}N_4O_4$, 367.14, found:367.1. HPLC > 99%.

**Example 2 Preparation of compounds B1 to B5**

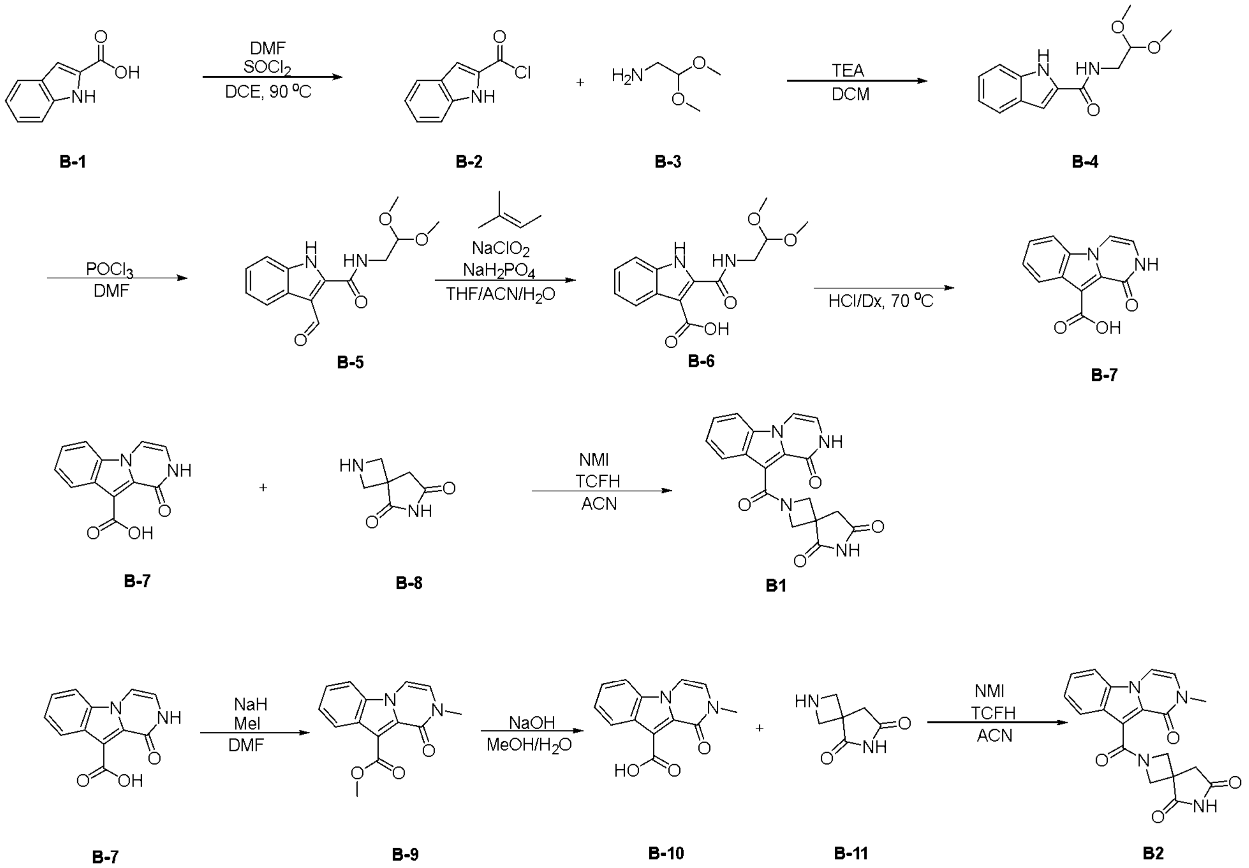


B-1 B-2 B-3 B-4

B-5 B-6 B-7

B-7 B-8 B1

B-7 B-9 B-10 B-11 B2

**Step 1 Synthesis of compound B-2**

**B-1** (5.00 g, 31.03 mmol), thionyl dichloride (9.24 g, 41.22 mmol) and dichloromethane (25 mL) were added into a 100 mL reaction flask. The reaction was heated at 90°C for 3 h until the reaction was completed. The reaction was quenched by adding water and extracted with ethyl acetate (50 mL×3). The organic phase was washed with concentrated brine, dried over anhydrous sodium sulfate and then filtered, and the solvent was evaporated to dryness to obtain 5.60 g of crude product **B-2**.

**Step 2 Synthesis of compound B-4**

**B-2** (5.60g, 31.18mmol), B-3 (3.28g, 62.36mmol), triethylamine (6.31 g, 62.36 mmol) and dichloromethane (20 mL) were added into a 50 mL reaction flask. The reaction was stirred at room temperature for 1 h until the reaction was completed. After vacuum distillation, the reactant was separated and purified by silica gel chromatography (petroleum ether:ethyl acetate=3:1) to obtain a light yellow solid **B-4** (4.50 g, 18.12 mmol, 58.13% yield).

**Step 3 synthesis of compound B-5**

To a 50 mL reaction flask, **B-4** (2.50 g, 10.07 mmol) and DMF (5 mL) were added, and phosphorus oxychloride (3.09 g, 20.14 mmol) dissolved in 15 mL DMF was added dropwise. The reaction was stirred at room temperature for 1 h until the reaction was completed. The reaction was quenched by adding water and extracted with ethyl acetate (50 mL×3). The organic phase was washed with concentrated brine, dried over anhydrous sodium sulfate and then filtered, and the solvent was evaporated to dryness to obtain 2.80 g of crude product **B-5**.

**Step 4 Synthesis of compound B-6**

To a 50 mL reaction flask, **B-5** (2.76 g, 10.00 mmol), 2-methyl-2-butene (3.51 g, 50.00 mmol), sodium dihydrogen phosphate (50.00 mmol) and a mixed solvent of acetonitrile/tetrahydrofuran/water (20 mL) were added, and disodium hydrogen phosphate (50.00 mmol) was added dropwise at 0°C. The reaction was stirred at room temperature for 12 h until the reaction was completed. The reaction solution was adjusted to pH=6.0 and extracted with ethyl acetate (50 mL×3). The organic phase was washed with concentrated brine, dried over anhydrous sodium sulfate and then filtered, and the solvent was evaporated to dryness to obtain 2.00 g of crude product **B-6**.

**Step 5 Synthesis of compound B-7**

B-6 (2.00 g, 6.84 mmol) was added into a 50 mL reaction flask and dissolved in 20 mL of HCl/dioxane (4M). The reaction was stirred at 70°C for 3 h until the reaction was completed. The solvent was evaporated to dryness, and the reactant was purified by MPLC to obtain **B-7** (0.30 g, 1.31 mmol, 19.21% yield).

**Step 6 Synthesis of compound B1**

TCFH (7.58 mg, 210.34 μmol) and NMI (350.56 μmol) were added into a 15 mL reaction flask, then dissolved in 5 mL of DMF at 0°C and activated for 0.2 h, and then **B-7** (40.00 mg, 175.28 μmol) and **B-8** (24.56 mg, 175.28 μmol) were added at room temperature, and reacted for 2 hours at room temperature until the reaction was completed. The solvent was evaporated to dryness, and the reactant was purified by MPLC to obtain **B1** (6.90 mg, 19.46 μmol, 11.10% yield, 98.8% purity) $^1$H NMR (600 MHz, DMSO-$d_6$) δ 11.20 (s, 1H), 11.00 (d, *J* = 5.5 Hz, 1H), 8.11 (d, *J* = 8.5 Hz, 1H), 7.90 (d, *J* = 5.6 Hz, 1H), 7.79 (d, *J* = 8.1 Hz, 1H), 7.47 (t, *J* = 7.5 Hz, 1H), 7.38 (t, *J* = 7.5 Hz, 1H), 6.77 (t, *J* = 5.6 Hz, 1H), 4.36-3.83 (m, 4H), 3.06 and 3.03 (s, 1H), 2.90 and 2.87 (s, 1H). LC-MS: $[M+H]^+$ $C_{18}H_{14}N_4O_4$, 351.1, found: 351.2. HPLC > 98%.

**Step 7 Synthesis of compound B-9**

To a 15 mL reaction flask, B-7 (0.20 g, 0.88 mmol), iodomethane (0.37 g, 2.64 mmol) and DMF (4 mL) were added, and sodium hydride (63.10 mg, 2.63 mmol) was added slowly at 0°C. The reaction was stirred at room temperature for 2 h until the reaction was completed. The reaction was quenched by adding water and extracted with ethyl acetate (50 mL×3). The organic phase was washed with concentrated brine, dried over anhydrous sodium sulfate and then filtered, and the solvent was evaporated to dryness to obtain 0.22 g of crude product **B-9**.

**Step 8 Synthesis of compound B-10**

To a 15 mL reaction flask, **B-9** (220.00 mg, 858.52 μmol) and a mixed solution of methanol/water (8 mL) were added, and sodium hydroxide (103.02 mg, 2.58 mmol) was added slowly. The reaction was stirred at 50°C for 2 h until the reaction was completed. The reaction solution was adjusted to pH=6.0 and extracted with ethyl acetate (50 mL×3). The organic phase was washed with concentrated brine, dried over anhydrous sodium sulfate and then filtered, and the solvent was evaporated to dryness to obtain 150.00 mg of crude product **B-10**.

**Step 9 Synthesis of compound B2**

To a 15 mL reaction flask, TCFH (8.93 mg, 247.70 μmol) and NMI (412.83 μmol) were added, and then dissolved in 3 mL of DMF at 0°C and activated for 0.2 h, and then **B-10** (50.00 mg, crude product) and **B-8** (28.93 mg, 206.42 μmol) were added at room temperature, and reacted for 2 hours at room temperature until the reaction was completed. The solvent was evaporated to dryness and the reactant was purified by MPLC to obtain B2 (26.60 mg, 71.98 μmol, 34.87% yield, 98.6% purity) $^1$H NMR (600 MHz, DMSO-*d*$_6$) δ 11.20 (s, 1H), 8.11 (d, *J* = 8.4 Hz, 1H), 7.99 (d, J = 5.9 Hz, 1H), 7.78 (d, J = 8.1 Hz, 1H), 7.55-7.33 (m, 2H), 7.02 (d, J = 5.9 Hz, 1H), 4.32-3.85 (m, 4H), 3.21 (s, 1H), 3.07 and 3.04 (s, 1H), 2.91 and 2.88 (s, 1H). LC-MS: [M+H]$^+$ $C_{19}H_{17}N_4O_4$, 365.1, found: 365.1. HPLC > 98%.

Referring to the synthesis method of compounds **B1** and **B2,** compounds **B3, B4** and **B5** can be obtained by replacing compound **B-3** with the raw materials in the following table 3, and keeping the other raw materials and operation methods unchanged.

| Compound No. | Data of structure and characterization of compounds | Raw materials | Yield |
|---|---|---|---|
| **B3** | 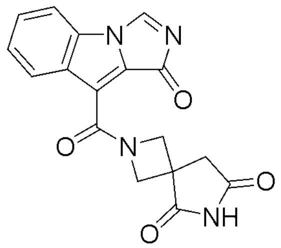<br>**B3**<br>Compound **B3** (5.72 mg, 13.13 μmol, purity 99.3%)。LC-MS: $C_{17}H_{13}N_4O_4$, [M+H]$^+$ 337.1; Found 337.0 $^1$H NMR (600 MHz, DMSO-*d*$_6$) δ 11.25 (s, 1H), 8.52 (s, 1H), 8.11 (d, *J* = 8.4 Hz, 1H), 7.78 (d, *J* = 8.1 Hz, 1H), 7.52-7.28 (m, 2H), 4.22-3.65 (m, 4H), 3.05 and 3.02 (s, 1H), 2.92 and 2.89 (s, 1H). | 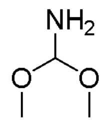<br>**B-11** | 23.21% |
| **B4** | 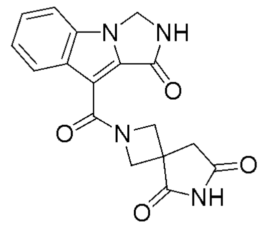<br>**B4**<br>Compound **B4** (4.26 mg, 12.59 μmol, purity 98.1%)。LC-MS: $C_{17}H_{15}N_4O_4$, [M+H]$^+$ 339.1; Found 339.2. $^1$H NMR (600 MHz, DMSO-*d*$_6$) δ 11.25 (s, 1H), 8.02 (d, *J* = 8.4 Hz, 1H), 7.88 (d, *J* = 8.1 Hz, 1H), 7.62-7.33 (m, 2H), 5.52 (s, 2H), 4.22-3.65 (m, 4H), 3.01 and 2.98 (s, 1H), 2.85 and 2.81(s, 1H). | 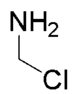<br>**B-12** | 18.22% |

(continued)

| Compound No. | Data of structure and characterization of compounds | Raw materials | Yield |
|---|---|---|---|
| B5 | 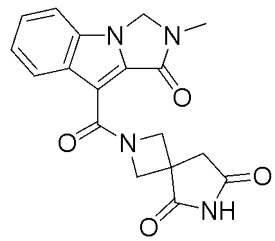 **B5** Compound **B5** (8.83 mg, 13.13 μmol, purity 99.5%)。 LC-MS: $C_{18}H_{17}N_4O_4$, $[M+H]^+$ 353.1 found 353.1. $^1H$ NMR (600 MHz, DMSO-$d_6$) δ 11.23 (s, 1H), 8.11 (d, $J$ = 8.2 Hz, 1H), 7.83 (d, $J$ = 8.3 Hz, 1H), 7.55-7.26 (m, 2H), 5.44 (s, 2H), 4.23-3.46 (m, 4H), 3.15 (s, 3H), 3.03 and 3.01 (s, 1H), 2.82 and 2.80 (s, 1H). | 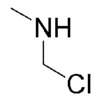 **B-13** | 37.72% |

In the present disclosure, the technical effects of the present disclosure are illustrated through the following test examples:

**Test example 1 Detection of inhibition of CRBN/DDB1 activity by compounds (FRET)**

1. Experimental materials and reagents

Microplate reader (BMG PHERAstar FSX), ECHO (LABCYTE Echo 665), microplate thermostatic oscillator (Hangzhou Ruicheng Instrument Co., Ltd.), disodium hydrogen phosphate (Sigma), sodium dihydrogen phosphate (Sigma), bovine serum albumin (Sigma), Anti-6His-Tb crypate Gold (CISBIO), CRBN/DDB1 protein (HitGen) and 384-well plate (Grenier Bio-one).

2. Experimental methods

The dry powder of the compound was dissolved in DMSO, and the compound was serially diluted using ECHO, and added to a 384-well reaction plate, so that the final concentration of DMSO in the whole reaction system (10.0 μL) was 1.0%, and the same amount of DMSO was added as a control.

20 mM disodium hydrogen phosphate, 20 mM sodium dihydrogen phosphate, 0.08% bovine serum albumin and buffer with pH of 7.0 were used to dilute the CRBN/DDB1 protein to twice the required final concentration (5.0 nM), and 5.0 μL of the diluted CRBN/DDB1 protein was added into the 384-well reaction plate added with compounds, centrifuged at 1000 rpm for 1 min, then placed on a microplate thermostatic oscillator, and pre-incubated at 25°C and 250 rpm for 15 min. 20 mM disodium hydrogen phosphate, 20 mM sodium dihydrogen phosphate, 0.08% bovine serum albumin and buffer with a pH of 7.0 were used to dilute Anti-6His-Tb crypate Gold and FITC-labeled thalidomide analogues to twice the required final concentration, and the final concentration of Anti-6His-Tb crypate Gold was 0.2 nM, the final concentration of FITC-labeled thalidomide analogues was 50.0 nM, and a mixed solution of Anti-6His-Tb crypate Gold/FITC-labeled thalidomide analogues was obtained. 5.0 μL of the mixed solution of Anti-6HIS-TB Crypate Gold/FITC-labeled thalidomide analogues was added into a 384-well reaction plate, centrifuged at 1000 rpm for 1 min, then placed on a microplate thermostatic oscillator, and incubated at 25°C and 250 rpm for 30 min. After the reaction was completed, the fluorescence signal value (Ex=337 nm Em=520/490 nm) in the 384-well reaction plate was read by the microplate reader.

3. Data analysis

The solvent group (containing 5.0 nM CRBN/DDB1, 0.2 nM Anti-6His-Tb crypate Gold, 50.0 nM FITC labeled thalidomide analogues and 1.0% DMSO) was used as the negative control and the reaction buffer group (containing 0.2 nM Anti-6His-Tb crypate Gold, 50.0 nM FITC labeled thalidomide analogues and 1.0% DMSO) was used as the blank control.

The percentage of residual activity of each concentration was calculated as follows:

Residual activity (%)=100%×(Flu $_{compound\ group}$ - Flu $_{blank\ control}$) /(Flu $_{negative\ control}$ -Flu $_{blank\ control}$)

Then, the dose-effect curve was fitted using GraphPad Prism 6.0 and the IC50 value was calculated accordingly..

Table 1: table of inhibition of CRBN/DDB1 protein by compounds

| No. | $IC_{50}$ (μM) |
|---|---|
| A1 | 0.009 |
| B1 | 0.052 |
| B2 | 0.024 |
| B3 | 0.043 |
| B4 | 0.018 |
| B5 | 0.011 |

The above experimental data show that the compounds of the present disclosure have a good inhibitory effect on CRBN/DDB1 protein and may become a new CRBN inhibitor medicine. Alternatively, the compound of the present disclosure may be used as an effective CRBN ligand, which can be used to further synthesize a corresponding PROTACs bifunctional compound that can degrade protein and target chimera.

## Claims

**1.** A compound represented by formula I, or a stereoisomer, or a deuterated compound, or a pharmaceutically acceptable salt thereof:

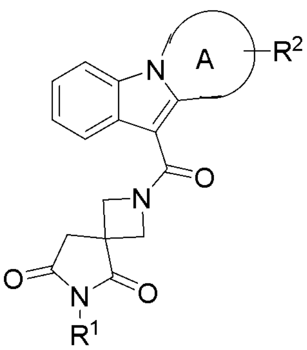

Formula I

wherein,

$R^1$ is selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-(3- to 10-membered cycloalkyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered heteroaromatic ring);

ring A is selected from the group consisting of 4- to 12-membered heterocycloalkyl and 5- to 10-membered heteroaromatic ring; wherein the heterocycloalkyl or heteroaromatic ring is further optionally substituted by one, two, three or four independent $R^{A1}$;

each $R^{A1}$ is independently selected from the group consisting of hydrogen, halogen, cyano, nitro, =O, =S, $=CR^{A2}R^{A3}$, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{A2}$, $-C_{0-4}$ alkylene-$OC(O)R^{A2}$, $-C_{0-4}$ alkylene-$SR^{A2}$, $-C_{0-4}$ alkylene-$S(O)_2R^{A2}$, $-C_{0-4}$ alkylene-$S(O)R^{A2}$, $-C_{0-4}$ alkylene-$S(O)_2NR^{A2}R^{A3}$, $-C_{0-4}$ alkylene-$S(O)NR^{A2}R^{A3}$, $-C_{0-4}$ alkylene-$C(O)R^{A2}$, $-C_{0-4}$ alkylene-$C(O)OR^{A2}$, $-C_{0-4}$ alkylene-$C(O)NR^{A2}R^{A3}$, $-C_{0-4}$ alkylene-$NR^{A2}R^{A3}$, $-C_{0-4}$ alkylene-$NR^{A2}C(O)R^{A3}$, $-C_{0-4}$ alkylene-$NR^{A2}S(O)_2R^{A3}$, $-C_{0-4}$ alkylene-$NR^{A2}S(O)R^{A3}$, $-C_{0-4}$ alkylene-(3- to 10-

membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring is further optionally substituted by one, two, three or four independent $R^{A4}$;

each $R^{A4}$ is independently selected from the group consisting of hydrogen, halogen, cyano, nitro, =O, =S, =$CR^{A2}R^{A3}$, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-4}$ alkylene-$OR^{A2}$, -$C_{0-4}$ alkylene-$OC(O)R^{A2}$, -$C_{0-4}$ alkylene-$SR^{A2}$, -$C_{0-4}$ alkylene-$S(O)_2R^{A2}$, -$C_{0-4}$ alkylene-$S(O)R^{A2}$, -$C_{0-4}$ alkylene-$S(O)_2NR^{A2}R^{A3}$, -$C_{0-4}$ alkylene-$S(O)NR^{A2}R^{A3}$, -$C_{0-4}$ alkylene-$C(O)R^{A2}$, -$C_{0-4}$ alkylene-$C(O)OR^{A2}$, -$C_{0-4}$ alkylene-$C(O)NR^{A2}R^{A3}$, -$C_{0-4}$ alkylene-$NR^{A2}R^{A3}$, -$C_{0-4}$ alkylene-$NR^{A2}C(O)R^{A3}$, -$C_{0-4}$ alkylene-$NR^{A2}S(O)_2R^{A3}$ and -$C_{0-4}$ alkylene-$NR^{A2}S(O)R^{A3}$;

$R^{A2}$ and $R^{A3}$ are independently selected from the group consisting of hydrogen, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl;

$R^2$ is selected from the group consisting of hydrogen, halogen, cyano, nitro, =O, =S, =$CR^{21}R^{22}$, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-4}$ alkylene-$OR^{21}$, -$C_{0-4}$ alkylene-$OC(O)R^{21}$, -$C_{0-4}$ alkylene-$SR^{21}$, -$C_{0-4}$ alkylene-$S(O)_2R^{21}$, -$C_{0-4}$ alkylene-$S(O)R^{21}$, -$C_{0-4}$ alkylene-$S(O)_2NR^{21}R^{22}$, -$C_{0-4}$ alkylene-$S(O)NR^{21}R^{22}$, -$C_{0-4}$ alkylene-$C(O)R^{21}$, -$C_{0-4}$ alkylene-$C(O)OR^{21}$, -$C_{0-4}$ alkylene-$C(O)NR^{21}R^{22}$, -$C_{0-4}$ alkylene-$NR^{21}R^{22}$, -$C_{0-4}$ alkylene-$NR^{21}C(O)R^{22}$, -$C_{0-4}$ alkylene-$NR^{21}S(O)_2R^{22}$, -$C_{0-4}$ alkylene-$NR^{21}S(O)R^{22}$, -$C_{0-4}$ alkylene-(3- to 10-membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring is further optionally substituted by one, two, three or four independent $R^{23}$;

each $R^{23}$ is independently selected from the group consisting of hydrogen, halogen, cyano, nitro, =O, =S, =$CR^{21}R^{22}$, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-4}$ alkylene-$OR^{21}$, -$C_{0-4}$ alkylene-$OC(O)R^{21}$, -$C_{0-4}$ alkylene-$SR^{21}$, -$C_{0-4}$ alkylene-$S(O)_2R^{21}$, -$C_{0-4}$ alkylene-$S(O)R^{21}$, -$C_{0-4}$ alkylene-$S(O)_2NR^{21}R^{22}$, -$C_{0-4}$ alkylene-$S(O)NR^{21}R^{22}$, -$C_{0-4}$ alkylene-$C(O)R^{21}$, -$C_{0-4}$ alkylene-$C(O)OR^{21}$, -$C_{0-4}$ alkylene-$C(O)NR^{21}R^{22}$, -$C_{0-4}$ alkylene-$NR^{21}R^{22}$, -$C_{0-4}$ alkylene-$NR^{21}C(O)R^{22}$, -$C_{0-4}$ alkylene-$NR^{21}S(O)_2R^{22}$, -$C_{0-4}$ alkylene-$NR^{21}S(O)R^{22}$, -$C_{0-4}$ alkylene-(3- to 10-membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring is further optionally substituted by one, two, three or four independent $R^{26}$;

$R^{21}$ and $R^{22}$ are independently selected from the group consisting of hydrogen, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-4}$ alkylene-$OR^{24}$, -$C_{0-4}$ alkylene-$OC(O)R^{24}$, -$C_{0-4}$ alkylene-$SR^{24}$, -$C_{0-4}$ alkylene-$S(O)_2R^{24}$, -$C_{0-4}$ alkylene-$S(O)R^{24}$, -$C_{0-4}$ alkylene-$S(O)_2NR^{24}R^{25}$, -$C_{0-4}$ alkylene-$S(O)NR^{24}R^{25}$, -$C_{0-4}$ alkylene-$C(O)R^{24}$, -$C_{0-4}$ alkylene-$C(O)OR^{24}$, -$C_{0-4}$ alkylene-$C(O)NR^{24}R^{25}$, -$C_{0-4}$ alkylene-$NR^{24}R^{25}$, -$C_{0-4}$ alkylene-$NR^{24}C(O)R^{25}$, -$C_{0-4}$ alkylene-$NR^{24}S(O)_2R^{25}$, -$C_{0-4}$ alkylene-$NR^{24}S(O)R^{25}$, -$C_{0-4}$ alkylene-(3- to 10-membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring is further optionally substituted by one, two, three or four independent $R^{26}$;

each $R^{26}$ is independently selected from the group consisting of hydrogen, halogen, cyano, nitro, =O, =S, =$CR^{24}R^{25}$, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-4}$ alkylene-$OR^{24}$, -$C_{0-4}$ alkylene-$OC(O)R^{24}$, -$C_{0-4}$ alkylene-$SR^{24}$, -$C_{0-4}$ alkylene-$S(O)_2R^{24}$, -$C_{0-4}$ alkylene-$S(O)R^{24}$, -$C_{0-4}$ alkylene-$S(O)_2NR^{24}R^{25}$, -$C_{0-4}$ alkylene-$S(O)NR^{24}R^{25}$, -$C_{0-4}$ alkylene-$C(O)R^{24}$, -$C_{0-4}$ alkylene-$C(O)OR^{24}$, -$C_{0-4}$ alkylene-$C(O)NR^{24}R^{25}$, -$C_{0-4}$ alkylene-$NR^{24}R^{25}$, -$C_{0-4}$ alkylene-$NR^{24}C(O)R^{25}$, -$C_{0-4}$ alkylene-$NR^{24}S(O)_2R^{25}$, -$C_{0-4}$ alkylene-$NR^{24}S(O)R^{25}$, -$C_{0-4}$ alkylene-(3- to 10-membered cycloalkyl), -$C_{0-4}$ alkylene-(4- to 10-membered heterocycloalkyl), -$C_{0-4}$ alkylene-(6- to 10-membered aromatic ring) and -$C_{0-4}$ alkylene-(5- to 10-membered heteroaromatic ring); wherein the alkylene, cycloalkyl, heterocycloalkyl, aromatic ring or heteroaromatic ring is further optionally substituted by one, two, three or four independent $R^{27}$;

$R^{24}$ and $R^{25}$ are independently selected from the group consisting of hydrogen, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl; and

each $R^{27}$ is independently selected from the group consisting of hydrogen, halogen, cyano, nitro, =O, =S, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl.

**2.** The compound according to claim 1, wherein $R^1$ is selected from the group consisting of hydrogen and -$C_{1-3}$ alkyl.

**3.** The compound according to claim 1, wherein $R^2$ is selected from the group consisting of hydrogen, halogen, cyano, nitro, =O, =S, -$C_{1-3}$ alkyl, -$C_{2-6}$ alkenyl, -$C_{2-6}$ alkynyl, halogen-substituted -$C_{1-3}$ alkyl, halogen-substituted -$C_{2-6}$ alkenyl and halogen-substituted -$C_{2-6}$ alkynyl.

**4.** The compound according to claim 1, wherein the ring A is selected from the group consisting of 5-membered heterocycloalkyl and 6-membered heterocycloalkyl; wherein the heterocycloalkyl is further optionally substituted by one, two, three or four independent $R^{A1}$; and
each $R^{A1}$ is independently selected from the group consisting of hydrogen, halogen, cyano, nitro, =O, -$C_{1-3}$ alkyl, -$C_{2-6}$ alkenyl, -$C_{2-6}$ alkynyl, halogen-substituted -$C_{1-3}$ alkyl, halogen-substituted -$C_{2-6}$ alkenyl and halogen-substituted -$C_{2-6}$ alkynyl.

**5.** The compound according to claim 4, wherein the ring A is selected from the group consisting of

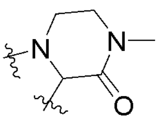, 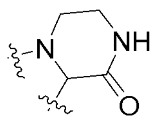, 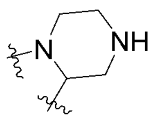, 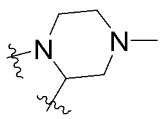, 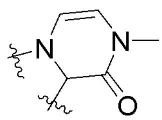,

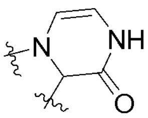, 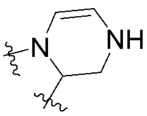, 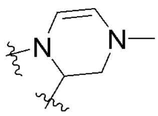, 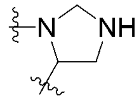, 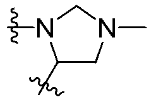, 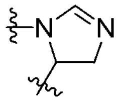, 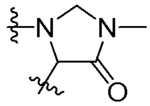,

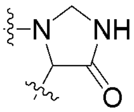 and 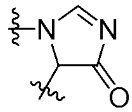.

**6.** The compound according to claim 1, wherein the compound is represented by formula IIA, formula IIB or formula IIC:

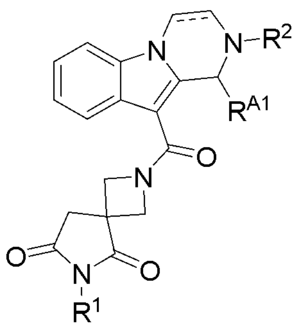

IIA

,

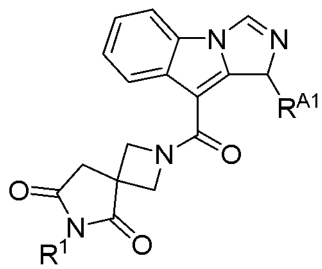

IIB

or

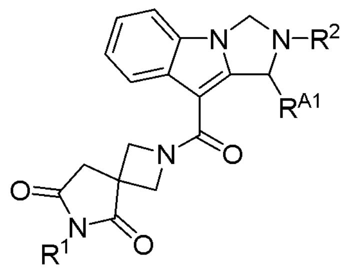

IIC

wherein,

$\overline{- - -}$ is selected from the group consisting of a single bond and a double bond; and
the substituents of $R^1$, $R^{A1}$ and $R^2$ are defined as in claim 1.

**7.** The compound according to any one of claims 1 to 6, wherein the compound is selected from the group consisting of

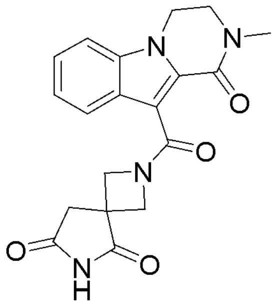,
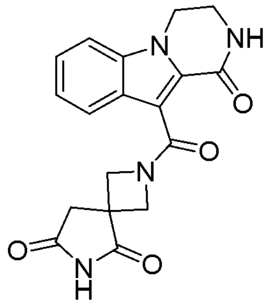,

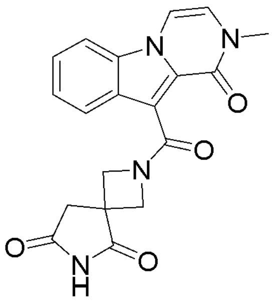,
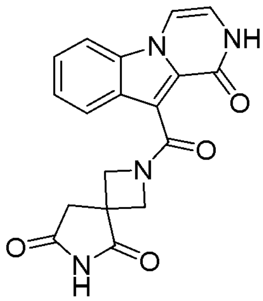,
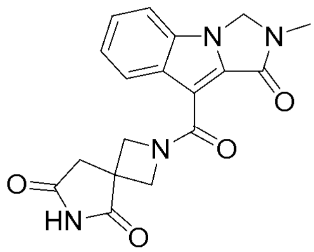,

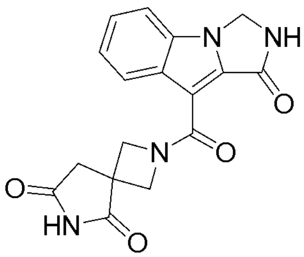 and 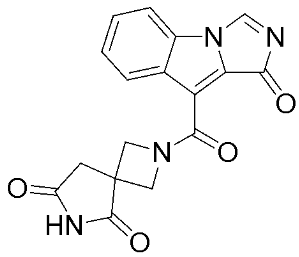.

**8.** Use of the compound according to any one of claims 1 to 7, or the stereoisomer thereof, or the deuterated compound thereof, or the pharmaceutically acceptable salt thereof in the manufacture of a medicament for treating a disease associated with abnormal proliferation of cells.

**9.** The use according to claim 8, wherein the disease is cancer.

**10.** Use of the compound according to any one of claims 1 to 7, or the stereoisomer thereof, or the deuterated compound thereof, or the pharmaceutically acceptable salt thereof in the manufacture of a medicament for degrading a target protein.

**11.** Use of the compound according to any one of claims 1 to 7, or the stereoisomer thereof, or the deuterated compound thereof, or the pharmaceutically acceptable salt thereof as an intermediate in the manufacture of a medicament for degrading a target protein.

**INTERNATIONAL SEARCH REPORT**

International application No.
**PCT/CN2024/076332**

**A. CLASSIFICATION OF SUBJECT MATTER**

C07D487/10(2006.01)i; A61K31/407(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D487/-,A61K31/-

Documentation searched other than minimum documentation to the extent that such documents are included in the fields searched

Electronic data base consulted during the international search (name of data base and, where practicable, search terms used)

CNTXT, CNKI, VEN, STN(REG, CAPLUS): 螺环, 小脑蛋白, 癌, cereblon, CRBN, spiro, cancer. 根据式I进行了结构检索, structure search according to formula I

**C. DOCUMENTS CONSIDERED TO BE RELEVANT**

| Category* | Citation of document, with indication, where appropriate, of the relevant passages | Relevant to claim No. |
|---|---|---|
| A | CN 112312904 A (C4 THERAPEUTICS INC.) 02 February 2021 (2021-02-02) entire document | 1-11 |
| A | CN 112566886 A (C4 THERAPEUTICS INC.) 26 March 2021 (2021-03-26) entire document | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 May 2024** | **28 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/076332**

| Patent document cited in search report | Publication date (day/month/year) | Patent family member(s) | Publication date (day/month/year) |
|---|---|---|---|
| CN 112312904 A | 02 February 2021 | US 2023416251 A1 | 28 December 2023 |
| | | WO 2019204354 A1 | 24 October 2019 |
| | | EP 3781156 A1 | 24 February 2021 |
| | | EP 3781156 A4 | 18 May 2022 |
| | | US 2021032245 A1 | 04 February 2021 |
| | | US 11584748 B2 | 21 February 2023 |
| CN 112566886 A | 26 March 2021 | WO 2019236483 A1 | 12 December 2019 |
| | | US 2021070763 A1 | 11 March 2021 |
| | | US 11623929 B2 | 11 April 2023 |
| | | EP 3802467 A1 | 14 April 2021 |
| | | EP 3802467 A4 | 13 April 2022 |
| | | EP 3578561 A1 | 11 December 2019 |
| | | US 2024018156 A1 | 18 January 2024 |

Form PCT/ISA/210 (patent family annex) (July 2022)